Europäisches Patentamt

European Patent Office

Office européen des brevets

(19)

(11) EP 0 757 044 B2

(12) **NEUE EUROPÄISCHE PATENTSCHRIFT**

(45) Veröffentlichungstag und Bekanntmachung des Hinweises auf die Entscheidung über den Einspruch:
**07.04.2004 Patentblatt 2004/15**

(45) Hinweis auf die Patenterteilung:
**19.08.1998 Patentblatt 1998/34**

(21) Anmeldenummer: 96108386.2

(22) Anmeldetag: **25.05.1996**

(51) Int Cl.$^7$: **C07D 301/12**, B01J 29/04, B01J 38/48

(54) **Verfahren zur Regenerierung eines Katalysators**

Process for the regeneration of a catalyst

Procédé pour la regénération d'un catalyseur

(84) Benannte Vertragsstaaten:
**AT BE DE DK ES FI FR GB IT NL PT SE**

(30) Priorität: **01.08.1995 DE 19528220**

(43) Veröffentlichungstag der Anmeldung:
**05.02.1997 Patentblatt 1997/06**

(73) Patentinhaber:
 • **Degussa AG**
  **40474 Düsseldorf (DE)**
 • **Krupp Uhde GmbH**
  **44141 Dortmund (DE)**

(72) Erfinder: **Thiele, Georg, Dr.**
  **63452 Hanau (DE)**

(74) Vertreter: **Fleischer, Holm Herbert, Dr. et al**
  **Sternagel, Fleischer, Godemeyer & Partner**
  **Patentanwälte**
  **Braunsberger Feld 29**
  **51429 Bergisch Gladbach (DE)**

(56) Entgegenhaltungen:
**EP-A- 0 100 119**

• **JOURNAL OF CATALYSIS , Bd. 129, Nr. 1, Mai 1991, LONDON, Seiten 159-167, XP000577042 M. G. CLERICI ET AL.: "Synthesis of propylene Oxide from propylene and Hydrogen Peroxide Catalyzed by Titanium Silicalite"**
• **APPLIED CATALYSIS A: GENERAL, Bd. 99, 1993, AMSTERDAM, Seiten 71-84, XP000196238 J.A. MARTENS ET AL.: "Hydroxylation of phenol with hydrogen peroxide on EUROTS-1 catalyst"**
• **JOURNAL OF CATALYSIS , Bd. 140, Nr. 1, März 1993, LONDON, Seite 71-83 XP000562771 MARIO G. CLERICI ET AL.: "Epoxidation of lower Olefins with Hydrogen Peroxide and Titanium Silicalite"**
• **Atlas of Zedite Structure Types second Reversed Edition 1987 pages 100 and 146**

**Beschreibung**

Die Erfindung betrifft ein Verfahren zur Regenerierung eines Katalysators, insbesondere eines zur Herstellung von Epoxiden aus Olefinen und Wasserstoffperoxid als Katalysator verwendeten, Titanatome enthaltenden Zeoliths.

Es ist bekannt, Epoxide aus Olefinen herzustellen, indem das Olefin mit Wasserstoffperoxid in Gegenwart eines Titanatome enthaltenden Zeoliths als Katalysator zum Epoxid umgesetzt wird (EP-A 0 100 119). M. G. Clerici, G. Bellussi und U. Romano, J. Catal. 129 (1991) 159-167 lehren weiterhin, daß der titanhaltige Zeolith während der Verwendung als Katalysator zur Epoxidierung von Propylenoxid rasch an katalytischer Aktivität verliert. Für die Regenerierung der Aktivität des Katalysators beschreiben Clerici et al. zwei Möglichkeiten:

Einerseits kann der Zeolith durch Kalzinieren bei 550°C regeneriert werden, andererseits kann die katalytische Aktivität durch Waschen mit Lösungsmitteln bei erhöhter Temperatur, vorzugsweise mit Methanol oder dem zur Epoxidierung verwendeten Lösungsmittel, wiederhergestellt werden. Die Regenerierung durch Kalzinieren hat den Nachteil, daß der Katalysator dazu zunächst getrocknet, dann auf die hohe Kalziniertemperatur erhitzt und anschließend wieder abgekühlt werden muß, was zu einem erheblichen zusätzlichen Energiebedarf und apparativen Aufwand führt. Die Regenerierung durch Waschen mit einem Lösungsmittel ist ein langsamer Prozeß, der in der Regel wesentlich mehr Zeit als die eigentliche Epoxidierungsreaktion erfordert.

Es besteht somit die Aufgabe, ein Verfahren zur Regenerierung eines Katalysators zu finden, welches rasch und einfach durchgeführt werden kann.

Gegenstand der Erfindung ist ein Verfahren zur Regenerierung eines zur Herstellung von Epoxiden aus Olefinen und Wasserstoffperoxid als Katalysator verwendeten, Titanatome enthaltenden Zeoliths, welches dadurch gekennzeichnet ist, daß man die katalytische Aktivität des gebrauchten Katalysators durch Behandeln mit Wasserstoffperoxid in Abwesenheit von Olefinen regeneriert und den regenerierten Katalysator erneut zur Epoxidierung von Olefinen einsetzt.

Die Epoxidierung eines Olefins, vorzugsweise von Propylen, wird, wie in EP 100 119 beschrieben, durchgeführt, wobei vorzugsweise Methanol als Lösungsmittel eingesetzt wird. Der zur Epoxidierung gebrauchte Titanatome enthaltende Zeolithkatalysator, dessen katalytische Aktivität während der Reaktion ganz oder teilweise erschöpft wird, wird anschließend von der Reaktionsmischung abgetrennt und gegebenenfalls mit einem Lösungsmittel, vorzugsweise mit Wasser, gewaschen. Der gebrauchte Katalysator wird danach mit einer Lösung von Wasserstoffperoxid in einem Lösungsmittel, vorzugsweise Wasser, umgesetzt. Die Wasserstoffperoxid-Stoffmenge wird dabei so gewählt, daß sie mehr als das Zehnfache, vorzugsweise mehr als das Hundertfache, der im Katalysator verbliebenen Stoffmenge an Olefin und mehr als das Zweifache, vorzugsweise mehr als das Zehnfache, der im Katalysator enthaltenen Stoffmenge an Titan beträgt. Die Konzentration der zur Regenerierung verwendeten $H_2O_2$-Lösung kann zwischen 0,01 und 70 Gew.-%, vorzugsweise zwischen 1 und 45 Gew.-% gewählt werden. Die Regenerierung kann bei Temperaturen zwischen 0 und 200°C, vorzugsweise zwischen 40 und 120°C durchgeführt werden. In einer bevorzugten Ausführungsform der Erfindung kann die Regenerierung bei dem Siedepunkt der wäßrigen $H_2O_2$-Lösung durchgeführt werden. Die zur Regenerierung erforderliche Zeit variiert mit der angewandten Regeneriertemperatur und $H_2O_2$-Konzentration und kann so gewählt werden, daß der gewünschte Grad an Regenerierung erreicht wird.

Der Katalysator wird danach von der Regenerierlösung abgetrennt und gegebenenfalls mit einem Lösungsmittel, vorzugsweise Wasser, gewaschen. Er kann in die Epoxidierungsreaktion zurückgeführt werden.

Falls das in der Regenerierung verwendete Lösungsmittel auch in der Epoxidierungsreaktion eingesetzt wird, kann der Katalysator auch ohne vorherige Abtrennung zusammen mit der zur Regenerierung eingesetzten Wasserstoffperoxidlösung in die Epoxidierungsreaktion zurückgeführt werden.

Wenn nur eine teilweise Regenerierung des Katalysators gewünscht wird, kann die erfindungsgemäße Regenerierung auch so durchgeführt werden, daß die aus der Reaktionsmischung abgetrennte Katalysatormenge in zwei Teile geteilt wird, nur eine der Teilmengen mit Wasserstoffperoxid regeneriert wird und beide Teilmengen gemeinsam oder getrennt in die Epoxidierungsreaktion zurückgeführt werden.

Gemäß Erfindung kann als Katalysator Titansilicalit in Form einer Suspension, die wahlweise aus reinem Titansilicalit, Titansilicalit mit einem Bindemittel oder Titansilicalit auf einem organischen oder anorganischen Trägermaterial besteht, eingesetzt werden. Die Epoxidierung des Olefins, vorzugsweise von Propylen, wird entsprechend EP 100119 durchgeführt, wobei vorzugsweise Methanol als Lösungsmittel verwendet wird. Zur Durchführung der Epoxidierung können Rührkesselreaktoren, Schlaufenreaktoren, Blasensäulen, Strömungsrohre oder andere geeignete Reaktoren für Suspensionen sowie Kombinationen dieser Reaktoren eingesetzt werden, wobei die Wahl des Reaktortyps vom Fachmann entsprechend den gewählten Reaktionsbedingungen und dem zu epoxidierenden Olefin getroffen wird. Der Katalysator wird anschließend durch Filtration, Zentrifugation, Dekantieren oder ein anderes geeignetes Verfahren von der Reaktionsmischung abgetrennt und mit einem Lösungsmittel, vorzugsweise Wasser, gewaschen. Danach kann der Katalysator erneut in einer wäßrigen Wasserstoffperoxidlösung mit einer Konzentration zwischen 0,01 und 70 Gew.-%, vorzugsweise zwischen 1 und 45 Gew.-%, besonders bevorzugt zwischen 10 und 45 Gew.-%, suspendiert und die Suspension auf einer Temperatur zwischen 0 und 150°C, bevorzugt zwischen 40 und 120°C, gehalten werden. Die Reaktionszeit für die Regenerierung des Katalysators wird dabei so gewählt, daß der gewünschte Regenerierungsgrad, vorzugsweise zwischen 20 und 100 % der ursprünglichen katalytischen Aktivität, erreicht wird. Anschließend wird der erneut regenerierte Katalysator in die Epoxidierungsreaktion zurückgeführt, wobei die zur Regenerierung verwendete Wasserstoffperoxidlösung gegebenenfalls ganz oder teilweise abgetrennt wird. Bei der bevorzugten Verwendung von wäßrigen Wasserstoffperoxidlösungen mit einem Gehalt zwischen 10 und 45 Gew.-% zur Regenerierung kann die Suspension des Katalysators in der Regenerierlösung vorteilhaft ohne Abtrennung des Katalysators in die Epoxidierungsreaktion geführt werden, so daß das nicht zur Regenerierung verbrauchte Wasserstoffperoxid zur Epoxidierung des Olefins genutzt wird.

In einer weiteren bevorzugten Ausführungsform der Erfindung wird ein mit einem Bindemittel verformter Titansilicalit als Katalysator in Form eines Festbetts zur Epoxidierung des Olefins eingesetzt. In diesem Fall wird die Katalysatorpackung zur Epoxidierung, bis die Aktivität des Katalysators ganz oder teilweise erschöpft ist, verwendet. Die Katalysatorpackung wird danach gegebenenfalls mit einem Lösungsmittel, vorzugsweise Wasser, gewaschen. Anschließend wird eine wäßrige Wasserstoffperoxidlösung mit einem Gehalt zwischen 0,01 und 70 Gew.-%, vorzugsweise zwischen 1 und 40 Gew.-%, bei einer Temperatur zwischen 0 und 150°C, vorzugsweise zwischen 40 und 120°C, über die Katalysatorpackung, bis der gewünschte Regenerierungsgrad erreicht ist, geleitet. Die zur Regenerierung verwendete Wasserstoffperoxidlösung kann dabei vorteilhaft im Kreislauf über die Katalysatorpackung geführt werden. Die Katalysatorpackung wird danach, gegebenenfalls nach einer zwischengeschalteten Wäsche mit einem Lösungsmittel, wieder zur Epoxidierung des Olefins eingesetzt.

Das erfindungsgemäße Verfahren zur Herstellung von Epoxiden aus Olefinen und Wasserstoffperoxid mit Titanatome enthaltenden Zeolithkatalysatoren und einer Regenerierung des Katalysators durch Behandeln des Katalysators mit Wasserstoffperoxid in Abwesenheit des Olefins besitzt gegenüber dem bekannten Stand der Technik zur Regenerierung des Katalysators durch Kalzinieren oder durch Waschen mit Lösungsmitteln den Vorteil einer einfacheren, rascheren und deutlich besseren Regenerierung und Rückführung des Katalysators in die Epoxidierungsreaktion. Es erlaubt eine wirtschaftlichere Herstellung von Epoxiden aus Olefinen und Wasserstoffperoxid als das bekannte Verfahren.

Beispiele

**Beispiel 1**

*Bestimmung der katalytischen Aktivität von Titansilicalit in der Epoxidierung von Propylen*

In einem thermostatisierten Laborautoklav mit Begasungsrührer werden 1 g Titansilicalit in 300 ml Methanol bei 40°C unter Propylenatmosphäre vorgelegt und bei 3 bar Überdruck mit Propylen gesättigt. Danach werden unter Rühren 12,9 g 30 Gew.-% wäßrige Wasserstoffperoxidlösung in einer Portion zugegeben und die Reaktionsmischung bei 40°C und 3 bar gehalten, wobei über einen Druckregler Propylen nachdosiert wird, um den Verbrauch durch die Reaktion auszugleichen. In regelmäßigen Abständen werden über einen Filter Proben entnommen und der Wasserstoffperoxidgehalt der Reaktionsmischung durch Redoxtitration mit Cer(IV) sulfat-Lösung bestimmt. Die Auftragung von ln $(c/c_0)$ gegen die Zeit t, wobei c die gemessene $H_2O_2$-Konzentration zum Zeitpunkt t und $c_0$ die berechnete $H_2O_2$-Konzentration zum Beginn der Reaktion ist, ergibt eine Gerade. Aus der Steigung der Geraden wird mit der Beziehung $\frac{dc}{dt} = -k.c.c_{kat}$, worin $c_{kat}$ für die Katalysatorkonzentration in kg Katalysator je kg Reaktionsmischung steht, die Aktivitätskennzahl k bestimmt. Die Aktivitätskennzahl nimmt mit zunehmender katalytischer Aktivität des Katalysators in der Epoxidierung von Propylen zu.

**Beispiel 2**

*Katalytische Aktivität von frischem Titansilicalit*

Titansilicalit, hergestellt nach der Vorschrift in J.P. A. Martens et al., Appl. Catal. A 99 (1993) 71 - 84, wird wie in Beispiel 1 auf seine Aktivität überprüft. Es wird eine Aktivitätskennzahl von 18,9 min[-1] ermittelt.

**Beispiel 3**

*Aktivitätsverlust von Titansilicalit durch die Verwendung als Katalysator zur Epoxidierung von Propylen*

In einem thermostatisierten Laborautoklav mit Begasungsrührer werden 5 g Titansilicalit gemäß Beispiel 2 in 270 g Methanol bei 40°C unter Propylenatmosphäre vorgelegt und bei 3 bar Überdruck mit Propylen gesättigt. Dann wird unter Rühren eine Mischung aus 560 g 50 Gew.-% Wasserstoffperoxid und 3600 g Methanol mit einer Rate von 600 g/h zudosiert und gleichzeitig so viel Reaktionsmischung über ein Filter, daß das Gewicht des Reaktorinhalts konstant bleibt, entnommen. Der Katalysator wird dabei durch den Filter in dem Reaktor zurückgehalten. Während der Wasserstoffperoxiddosierung wird über einen Druckregler Propylen nachdosiert, um den Druck im Reaktor konstant zu halten. Nach 7 h wird die Wasserstoffperoxid-Dosierung abgebrochen, der Katalysator abfiltriert, bei 20°C mit Methanol gewaschen und bei 20°C an der Luft getrocknet. Der gebrauchte Katalysator wird gemäß Beispiel 1 auf seine Aktivität überprüft. Es wird eine Aktivitätskennzahl von 1,3 min$^{-1}$ bestimmt. Eine Wiederholung des Aktivitätstests nach 5 Tagen gemäß Beispiel 1 ergibt eine Aktivitätskennzahl von 1,7 min$^{-1}$. Die ermittelten Aktivitätskennzahlen von 1,3 bzw. 1,7 min$^{-1}$ zeigen, daß Titansilicalit bei der Verwendung als Katalysator zur Epoxidierung von Propylen mit Wasserstoffperoxid seine katalytische Aktivität für diese Reaktion weitgehend verliert und seine katalytische Aktivität ohne Maßnahmen zur Regenerierung nicht zurückgewinnt.

### Beispiel 4 und 5 (Vergleichsbeispiele)

*Regenerierung von gebrauchtem Titansilicalit durch Auskochen mit Methanol oder Wasser*

### Beispiel 4

1 g gebrauchter Titansilicalit-Katalysator aus Beispiel 3 wird 4 h in 25 ml Methanol unter Rückfluß erhitzt und anschließend filtriert. Die katalytische Aktivität wird gemäß Beispiel 1 überprüft. Es wird eine Aktivitätskennzahl von 1,3 min$^{-1}$ bestimmt.

### Beispiel 5

1 g gebrauchter Titansilicalit-Katalysator aus Beispiel 3 wird 4 h in 25 ml vollentsalztem Wasser unter Rückfluß erhitzt, anschließend filtriert und mit Methano gewaschen. Die katalytische Aktivität wir gemäß Beilspiel 1 überprüft. Es wird eine Aktivitätskennzahl von 1,6 min$^{-1}$ bestimmt.
Die Beispiele 4 und 5 zeigen, daß eine Regenerierung von gebrauchtem Titansilicalit durch Auskocher mit Methanol bei 65°C bzw. mit Wasser bei 100°C innerhalb von 4 h keine wesentliche Verbesserung der katalytischen Aktivität bewirkt.

### Beispiele 6 - 8 (gemäß Erfindung)

*Regenerierung von gebrauchtem Titansilicalit durch Auskochen mit Wasserstoffperoxid*

### Beispiel 6

1 g gebrauchter Titansilicalit-Katalysator aus Bei spiel 1 wird 4 h in 25 ml 5 Gew.-% wäßriger Wasserstoff peroxidlösung unter Rückfluß erhitzt, anschließend fil triert, mit Wasser und Methanol gewaschen. Die kataly tische Aktivität wird gemäß Beispiel 1 überprüft. Es wird eine Aktivitätskennzahl von 18,3 min$^{-1}$ bestimmt.

### Beispiel 7

Beispiel 6 wird wiederholt, wobei die Behandlung mit Wasserstoffperoxid auf 1 h verkürzt wird. Es wird eine Aktivitätskennzahl von 11,2 min$^{-1}$ bestimmt.

### Beispiel 8

Beispiel 6 wird wiederholt, wobei die Behandlung mit Wasserstoffperoxid auf 15 min verkürzt wird. Es wird eine Aktivitätskennzahl von 4,9 min$^{-1}$ bestimmt.
Die Beispiele 6 - 8 zeigen, daß sich gebrauchter Titansilicalit durch eine erfindungsgemäße Behandlung mit 5 Gew.-% Wasserstoffperoxid bei 100°C innerhalb von 4 h vollständig regenerieren läßt und daß durch die Wahl der Behandlungszeit mit Wasserstoffperoxid gezielt eine teilweise Regenerierung des Katalysators erreicht werden kann.

### Patentansprüche

1.  Verfahren zur Regenerierung eines zur Herstellung von Epoxiden aus Olefinen und Wasserstoffperoxid als Katalysator verwendeten, Titanatome enthaltenden Zeoliths, **dadurch gekennzeichnet, daß** man die katalytische Aktivität des gebrauchten Katalysators du rch Behandeln mit Wasserstoffperoxid in Abwesenheit von Olefinen regeneriert und den regenerierten Katalysator erneut zur Epoxidierung von Olefinen einsetzt.

2.  Verfahren gemäß Anspruch 1, **dadurch gekennzeichnet, daß** man den gebrauchten Katalysator mit einer wäßrigen Wasserstoffperoxidlösung, die zwischen 0,01 und 70 Gew.-% Wasserstoffperoxid enthält, regeneriert.

3.  Verfahren gemäß Anspruch 2, **dadurch gekennzeichnet, daß** man den gebrauchten Katalysator mit einer wäßrigen Wasserstoffperoxidlösung, die zwischen 1 und 45 Gew.-% Wasserstoffperoxid enthält, regeneriert.

4.  Verfahren gemäß den Ansprüchen 1 - 3, **dadurch gekennzeichnet, daß** man die Regenerierung bei einer Temperatur zwischen 0 und 200°C durchführt.

5.  Verfahren gemäß Anspruch 4, **dadurch gekennzeichnet, daß** man die Regenerierung bei einer Temperatur zwischen 40 und 120°C durchführt.

6.  Verfahren zur Herstellung eines Epoxids durch Umsetzung eines Olefins mit Wasserstoffperoxid in Gegenwart eines Titanatome enthaltenden Zeoliths als Katalysator, **dadurch gekennzeichnet, daß** man die katalytische Aktivität des gebrauchten Katalysators durch Behandein mit Wasserstoffperoxid in Abwesenheit des Olefins gemäß Anspruch 1 bis 5 regeneriert und den regenerierten Katalysator erneut zur Epoxidierung einsetzt.

7. Verfahren gemäß Anspruch 6, **dadurch gekennzeichnet, daß** man als Olefin Propylen einsetzt.

8. Verfahren gemäß den Ansprüchen 6 und 7, **dadurch gekennzeichnet, daß** man die Epoxidierungsreaktion in einer Mischung von Methanol und Wasser als Lösungsmittel durchführt.

9. Verfahren gemäß den Ansprüchen 6 - 8, **dadurch gekennzeichnet, daß** man den Katalysator in Form einer Suspension einsetzt.

10. Verfahren gemäß den Ansprüchen 6 - 9, **dadurch gekennzeichnet, daß** man den gebrauchten Katalysator mit einer wäßrigen Wasserstoffperoxidlösung, die zwischen 10 und 45 Gew.-% Wasserstoffperoxid enthält, regeneriert und die Mischung aus Katalysator und Wasserstoffperoxid von der Regenerierung ohne vorherige Trennung in die Epoxidierungsreaktion einsetzt.

11. Verfahren gemäß Anspruch 6, **dadurch gekennzeichnet, daß** man den Katalysator in Form eines Festbetts einsetzt.

12. Verfahren gemäß Anspruch 11, **dadurch gekennzeichnet, daß** man die Wasserstoffperoxidlösung während der Regenerierung im Kreislauf über das Katalysator-Festbett führt.

## Claims

1. Process for the regeneration of a titanium atom-containing zeolite catalyst used for the preparation of epoxides from olefines and hydrogen peroxide, **characterised in that** the catalytic activity of the used catalyst is regenerated by treatment with hydrogen peroxide in the absence of olefines, and the regenerated catalyst is utilised again for the epoxidisation of olefines.

2. Process according to Claim 1, **characterised in that** the used catalyst is regenerated with an aqueous hydrogen peroxide solution which contains between 0.01 and 70 wt.% hydrogen peroxide.

3. Process according to Claim 2, **characterised in that** the used catalyst is regenerated with an aqueous hydrogen peroxide solution which contains between 1 and 45 wt.% hydrogen peroxide.

4. Process according to Claims 1 to 3, **characterised in that** the regeneration is carried out at a temperature of between 0 and 200°C.

5. Process according to Claim 4, **characterised in that** the regeneration is carried out at a temperature of between 40 and 120°C.

6. Process for the preparation of an epoxide by reacting an olefine with hydrogen peroxide in the presence of a titanium atom-containing zeolite catalyst, **characterised in that** the catalytic activity of the used catalyst is regenerated by treatment with hydrogen peroxide in the absence of the olefine according to Claims 1 to 5, and the regenerated catalyst is utilised again for the epoxidisation.

7. Process according to Claim 6, **characterised in that** propylene is utilised as the olefine.

8. Process according to Claims 6 and 7, **characterised in that** the epoxidisation reaction is carried out in a mixture of methanol and water as a solvent.

9. Process according to Claims 6 to 8, **characterised in that** the catalyst is utilised in the form of a suspension.

10. Process according to Claims 6 to 9, **characterised in that** the used catalyst is regenerated with an aqueous hydrogen peroxide solution which contains between 10 and 45 wt.% hydrogen peroxide, and the mixture of catalyst and hydrogen peroxide from the regeneration is introduced into the epoxidisation reaction without prior separation.

11. Process according to Claim 6, **characterised in that** the catalyst is utilised in the form of a fixed bed.

12. Process according to Claim 11, **characterised in that** the hydrogen peroxide solution is circulated by way of the catalyst fixed bed during the regeneration.

## Revendications

1. Procédé pour la régénération d'une zéolithe contenant des atomes de titane utilisée en tant que catalyseur pour la préparation d'époxydes à partir d'oléfines et du peroxyde d'hydrogène, **caractérisé en ce que** l'on régénère l'activité catalytique du catalyseur usé par traitement au peroxyde d'hydrogène en absence d'oléfines et que l'on réutilise de nouveau le catalyseur régénéré pour l'époxydation des oléfines.

2. Procédé selon la revendication 1, **caractérisé en ce que** l'on régénère le catalyseur usé au moyen d'une solution aqueuse de peroxyde d'hydrogène contenant entre 0,01 et 70 % en poids de peroxyde d'hydrogène.

3. Procédé selon la revendication 2, **caractérisé en ce que** l'on régénère le catalyseur usé au moyen d'une solution aqueuse de peroxyde d'hydrogène contenant entre 1 et 45 % en poids de peroxyde d'hydrogène.

4. Procédé selon les revendications 1 à 3, **caractérisé en ce que** l'on réalise la régénération à une température entre 0 et 200° C.

5. Procédé selon la revendication 4, **caractérisé en ce que** l'on réalise la régénération à une température entre 40 et 120° C.

6. Procédé pour la production d'un époxyde par réaction d'une oléfine avec du peroxyde d'hydrogène en présence d'une zéolithe contenant des atomes de titane en tant que catalyseur, **caractérisé en ce que** l'on régénère l'activité catalytique du catalyseur usé par traitement au peroxyde d'hydrogène en absence d'oléfine selon les revendications 1 à 5 et que l'on réutilise de nouveau le catalyseur régénéré pour l'époxydation.

7. Procédé selon la revendication 6, **caractérisé en ce que** l'on utilise du propylène en tant qu'oléfine.

8. Procédé selon les revendications 6 et 7, **caractérisé en ce que** l'on réalise la réaction d'époxydation dans un mélange de méthanol et d'eau en tant que solvant.

9. Procédé selon les revendications 6 à 8, **caractérisé en ce que** l'on utilise le catalyseur sous la forme d'une suspension.

10. Procédé selon les revendications 6 à 9, **caractérisé en ce que** l'on régénère le catalyseur usé au moyen d'une solution aqueuse de peroxyde d'hydrogène contenant entre 10 et 45 % en poids de peroxyde d'hydrogène, et que l'on utilise le mélange constitué par le catalyseur et le peroxyde d'hydrogène provenant de la régénération, sans séparation préalable, dans la réaction d'époxydation.

11. Procédé selon la revendication 6, **caractérisé en ce que** l'on utilise le catalyseur sous la forme d'un lit fixe.

12. Procédé selon la revendication 11, **caractérisé en ce que** l'on recycle en boucle la solution de peroxyde d'hydrogène pendant la régénération sur le lit fixe du catalyseur.